# EUROPEAN PATENT APPLICATION

(11) **EP 3 533 381 A1**
(43) Date of publication of application: **04.09.2019**
(21) Application number: 17868934.5
(22) Date of filing: 12.10.2017
(51) Int. Cl.: A61B 1/045, A61B 1/00, G02B 23/24

(54) **CONTROL DEVICE FOR ENDOSCOPE SYSTEM AND CONTROL METHOD FOR ENDOSCOPE SYSTEM**

(30) Priority: 10.11.2016 JP 2016219523
(71) Applicant: Sony Corporation, Tokyo 108-0075 (JP); Sony Olympus Medical Solutions Inc., Tokyo 192-0904 (JP)
(72) Inventor: KOKUBO, Wataru, Tokyo 108-0075 (JP); FUKUSHIMA, Tetsuharu, Tokyo 108-0075 (JP); SUGAI, Toshiya, Hachioji-shi Tokyo 192-0904 (JP)
(74) Representative: D Young & Co LLP
(86) International application number: PCT/JP2017/037034
(87) International publication number: WO 2018/088107

(57) **Abstract**

[Object] To make the vertical and horizontal directions of an endoscope image and the vertical and horizontal directions of the screen of a monitor coincide with each other without spoiling the effective region of the endoscope image.

[Solution] A control device of an endoscope system according to the present disclosure includes: a scope rotation angle acquiring section that acquires a scope rotation angle, around an axis in an insertion direction, of a scope including an objective lens to be inserted in a body; and a rotation angle control section that controls a rotation angle, around an optical axis center, of an image sensor that captures an image provided by the scope, on the basis of the scope rotation angle.

## Description

### Technical Field

The present disclosure relates to a control device of an endoscope system and a control method of an endoscope system.

### Background Art

Hitherto, for example, in Patent Literature 1 described in the below, described is a technology that supposes to make it possible to display on a monitor an endoscope image in which the vertical and horizontal directions of the endoscope image coincide with the vertical and horizontal operation directions of a worker such that the worker does not feel uncomfortable at the time of working.

### Citation List

### Patent Literature

Patent Literature 1: JP 2006-218027A

### Disclosure of Invention

### Technical Problem

However, the technology disclosed in the above-described Patent Literature 1 is one that causes an endoscope display image to be rotated by image processing. Accordingly, in the case of trying to display a normally-rectangular endoscope image on a rectangular monitor by causing the endoscope image to be rotated by the image processing, the four corners of the endoscope image protrude to the outside of the upper, lower, right, and left frames of the monitor, which causes a problem that the effective display region of the endoscope display image is limited.

Moreover, the technology described in the above-described Patent Literature 1 is made to target a straightly-viewing scope, and has considered nothing about the endoscope image acquired by an obliquely-viewing scope that is suitable for observation with a wider viewing angle.

Then, it has been requested that the vertical and horizontal directions of an endoscope image and the vertical and horizontal directions of the screen of a monitor are made to coincide with each other without spoiling the effective region of the endoscope image.

### Solution to Problem

According to the present disclosure, there is provided a control device of an endoscope system, including: a scope rotation angle acquiring section that acquires a scope rotation angle, around an axis in an insertion direction, of a scope including an objective lens to be inserted in a body; and a rotation angle control section that controls a rotation angle, around an optical axis center, of an image sensor that captures an image provided by the scope, on the basis of the scope rotation angle.

In addition, according to the present disclosure, there is provided a control method of an endoscope system, including: acquiring a scope rotation angle, around an axis in an insertion direction, of a scope including an objective lens to be inserted in a body; and controlling a rotation angle, around an optical axis center, of an image sensor that captures an image provided by the scope, on the basis of the scope rotation angle.

In addition, according to the present disclosure, there is provided a control device of an endoscope system, including: a camera rotation angle calculating section that calculates a rotation angle, around an optical axis center, of an image sensor that captures an image provided by a scope including an objective lens to be inserted in a body; and a rotation angle control section that controls a rotation angle, around an optical axis center, of the image sensor on the basis of the rotation angle.

In addition, according to the present disclosure, there is provided a control method of an endoscope system, including: calculating a rotation angle, around an optical axis center, of an image sensor that captures an image provided by a scope including an objective lens to be inserted in a body; and controlling a rotation angle, around an optical axis center, of the image sensor on the basis of the rotation angle.

### Advantageous Effects of Invention

As described in the above, according to the present disclosure, it becomes possible to make the vertical and horizontal directions of an endoscope image and the vertical and horizontal directions of the screen of a monitor coincide with each other without spoiling the effective region of the endoscope image.

Note that the effects described above are not necessarily limitative. With or in the place of the above effects, there may be achieved any one of the effects described in this specification or other effects that may be grasped from this specification.

### Brief Description of Drawings

FIG. 1 is a perspective view showing a basic constitution of a system according to one embodiment of the present disclosure.
FIG. 2 is a schematic illustration showing the whole constitution including a system according to one embodiment of the present disclosure.
FIG. 3 is a schematic illustration for describing one example of a using method of a system according to one embodiment of the present disclosure.
FIG. 4 is a schematic illustration showing a combination of FIG. 1 and FIG. 3.
FIG. 5 is a schematic illustration showing a positional relationship in the case of viewing from an upper surface, a front surface (target organization side), and a back surface (camera side) of an obliquely-viewing scope.
FIG. 6 is a schematic illustration showing an enlarged illustration of a camera visual-field flat plane.
FIG. 7A a schematic illustration showing a scope image displayed on a monitor and a surgeon.
FIG. 7B a schematic illustration showing a scope image displayed on a monitor and a surgeon.
FIG. 7C a schematic illustration showing a scope image displayed on a monitor and a surgeon.
FIG. 8 is a schematic illustration showing a basic constitution in the case of using a straightly-viewing scope instead of an obliquely-viewing scope.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

It should be noted that description will be given in the following order.
1. Background
2. Basic constitution of system
3. Whole constitution including system
4. Using method of system
5. Constitution example of control device
6. Constitution example in case of straightly-viewing scope

### 1. Background

In order to secure the accuracy and easiness of a surgery in a general surgical operation, it is considered important to maintain good coordination (hereinafter, referred to as hand eye coordination) between visual sense and motions of hands. In particular, in a surgery that is performed while looking a video image, such as an endoscopic surgical operation, it is considered as a basic matter to reproduce the relationship between a visual line of a human body and the vector of a motion space of hands as much as possible. In particular, in the case where there is a divergence exceeding 90 degrees in motions of hands in a video image relative to a positional relationship of a human body, or discrepancies in the top and bottom of an image, it has been known that the surgery becomes difficult.

Especially, in the case of using an obliquely-viewing scope in which an objective optical system forms an angle relative to an eyepiece optics system of a scope, in order to secure good hand eye coordination, it is necessary to make the obliquely-viewing scope rotate around the optical axis, as the center, of a camera for observation and not to make the camera rotate. Such an operation for rotating the obliquely-viewing scope for observation is called obliquely-viewing rotation.

This operation is an operation performed in the case where a scope operator who performs operation for a scope and a camera, is operating while holding the scope and the camera, and it is requested to make the vertical direction (the vertical direction of an image displayed on a monitor) of the camera (image sensor) and the direction of the gravitational acceleration G coincide with each other always. Moreover, there may be a case where it is requested to make the vertical direction of the head (vertical direction relative to the horizontal plane connecting both eyes) of a surgeon and the vertical direction of a camera coincide with each other as required.

On the other hand, in the case where having used a video system of high image quality represented by 4K, or a 3D system, the influence that the hand shaking of a scope operator gives to a video image, has become considerably larger than the related video system. Accordingly, much more precise operation is requested for the scope operator. However, there is a limit in the operation quality capable of being achieved by a human, and there is a problem of fatigue of the scope operator due to surgery performed over long time. Moreover, there are problems caused by a matter that the scope operator grasps and operates, such as occurrence of the above-mentioned hand shaking derived from the above fatigue.

Therefore, it is considered to provide an image of high quality for a surgeon by releasing from hand shaking and fatigue over long time by grasping a scope and a camera with a holding device. However, in the case of using the holding device, it becomes difficult to perform the above-mentioned obliquely-viewing rotation.

### 2. Basic constitution of system

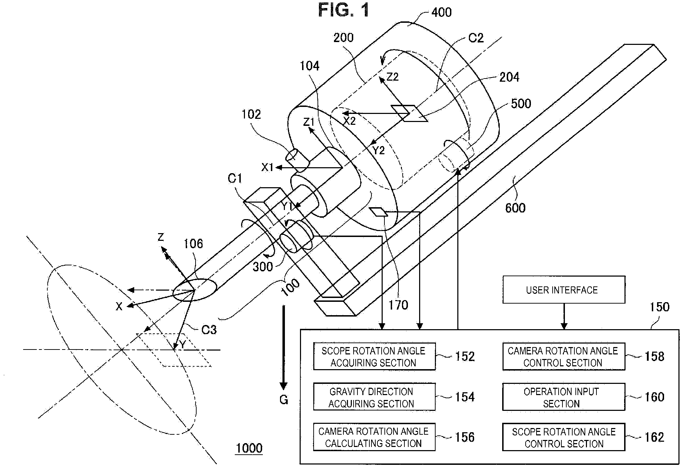

FIG. 1 is a perspective view showing a basic constitution of an endoscope system 1000 according to one embodiment of the present disclosure. A system according to the present embodiment includes an obliquely-viewing scope 100 and a camera 200. In the obliquely-viewing scope 100, its tip is inserted into the body of a patient. The camera 200 includes an image sensor 204 that photographs a scope image. The camera 200 includes an imaging optical system (not shown) on the front side of the image sensor 204.

The obliquely-viewing scope 100 includes a light guide connector 102 that receives illumination light from a light source device (not shown) and an eyepiece section 104 that is connected with the camera 200. The obliquely-viewing scope 100 and the camera 200 are connected such that the optical axis C1 of an eyepiece optics system of the obliquely-viewing scope 100 and the optical axis C2 of an imaging optical system of the camera 106 coincide with each other.

At a distal end of the obliquely-viewing scope 100, an objective lens section 106 is arranged such that the optical axis C1 of the eyepiece optics system and the optical axis C3 of the objective optical system form a predetermine angle.

Moreover, the obliquely-viewing scope 100 is supported by a holding section 600, and is made rotatable freely relative to the holding section 600. The obliquely-viewing scope 100 is made to rotate relative to the holding section 600 by a driving force of a scope rotating device 300.

The camera 200 is stored substantially integrally in the housing 400. The housing 400 is supported by the holding section 600. The camera 200 is made rotatable freely relative to the housing 400, and is driven relative to the housing 400 by a driving force of a camera rotating device 500. The scope rotating device 300 and the camera rotating device 500 include a motor, an angle sensor that detects the rotation angle of an output shaft of the motor, and the like.

In this connection, the scope rotating device 300 may be constituted so as to rotate the obliquely-viewing scope 100 manually.

In FIG. 1, each coordinate system is as described in the below. The distal direction of the optical axis C3 of the objective optical system is defined as the Y axis positive direction, the vertically upward direction (the approximately relative direction with respect to the gravity G) orthogonal to the Y axis on a flat plane of the objective lens section 106 vertical to the Y axis is defined as the Z axis positive direction, and the horizontally right side (the right side in the case of facing the Y axis positive direction) orthogonal to the Z axis on the same plane is defined as the X axis positive direction.

Moreover, the distal direction of the optical axis C1 of the eyepiece optic system is defined as the Y1 axis positive direction, the vertically upward direction (the approximately relative direction with respect to the gravity G) orthogonal to the Y1 axis on a flat plane of the eyepiece section 104 vertical to the Y1 axis is defined as the Z1 axis positive direction, and the horizontally right side (the right side in the case of facing the Y1 axis positive direction) orthogonal to the Z1 axis on the same plane is defined as the X1 axis positive direction.

Moreover, the distal direction of the imaging optical system disposed on the front side of the image sensor 204 is defined as the Y2 axis positive direction, the vertically upward direction (the approximately relative direction with respect to the gravity G) orthogonal to the Y2 axis on a flat plane (imaging surface) of the image sensor 204 vertical to the Y2 axis is defined as the Z2 axis positive direction, and the horizontally right side (the right side in the case of facing the Y2 axis positive direction) orthogonal to the Z1 axis on the same plane is defined as the X2 axis positive direction.

### 3. Whole constitution including system

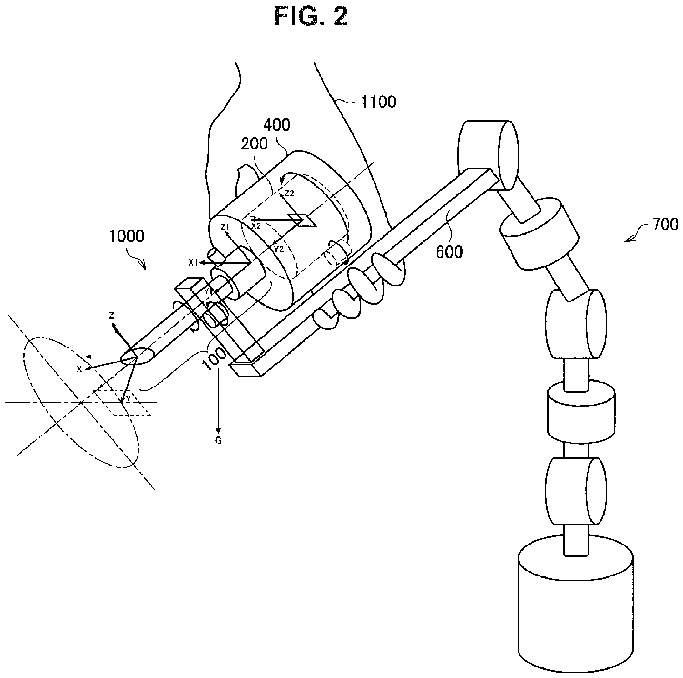

FIG. 2 is a schematic illustration showing the whole constitution including the endoscope system 1000 according to one embodiment of the present disclosure. As shown in FIG. 2, the holding section 600 may be supported by a supporting arm 700. As shown in FIG. 2, a user (surgeon 1100) can perform an operation by grasping the holding section 600 directly, and the supporting arm 700 may be bent electrically or manually, or with the other manner in response to an operation of the surgeon 1100, and, maintains the finally-bent state when the surgeon 1100 releases the hand. Moreover, the endoscope system 1000 also includes, in addition to the gravity sensor 170 and the control device 150 shown in FIG. 1, a motor control device that rotate the scope rotating device 300 and the camera rotating device 500, as a constitution element.

### 4. Using method of system

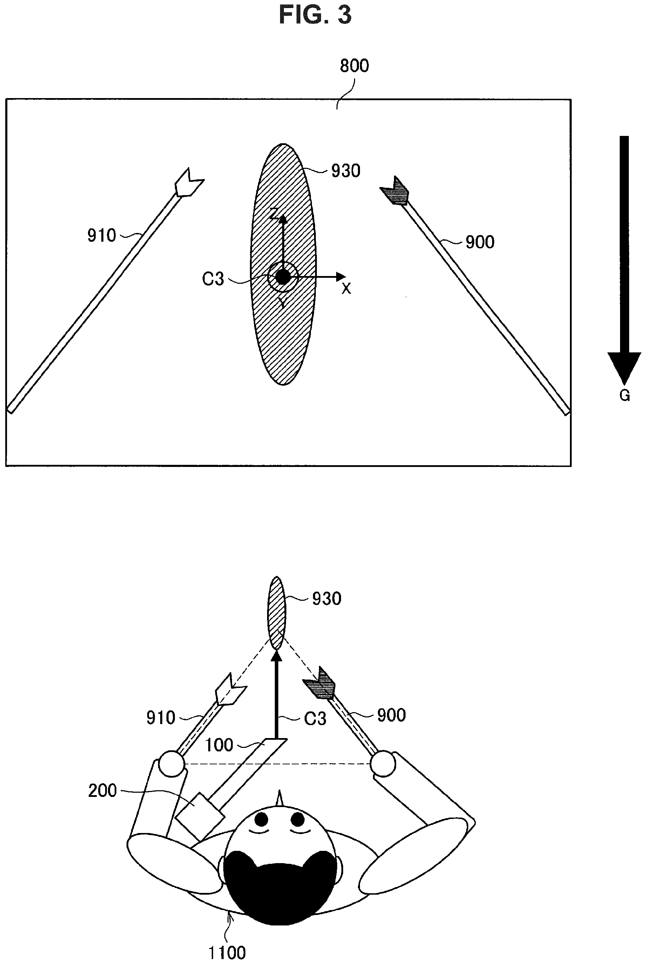

FIG. 3 is a schematic illustration for describing one example of a using method of the endoscope system 1000. The surgeon 1100 performs surgery while observing a scope image acquired by the obliquely-viewing scope 100 and the camera 200 on the screen of a monitor 800. On the monitor 800, an image (endoscope image) imaged by the image sensor 204 is displayed. The monitor 800 performs display by making the vertical direction of a screen and the direction of the gravitational acceleration G coincide with each other.

The surgeon 1100 holds a right hand forceps 900 with the right hand and a left hand forceps 910 with the left hand, arranges them so as to constitute an isosceles triangle in which a target organization 930 handled as a treatment purpose is placed at the vertex, oneself is placed at the base, and the right hand forceps 900 and the left hand forceps 910 are placed at the respective hypotenuses, and takes a viewing direction such that the optical axis C3 of the objective optical system of the obliquely-viewing scope 100 is positioned on the vertical bisector of the isosceles triangle, which is deemed as a basic manner. In the case of the positional relationship is formed in accordance with the basic manner, the hand eye coordination can be taken with a manner similar to that in the surgery at the time of straightly-viewing.

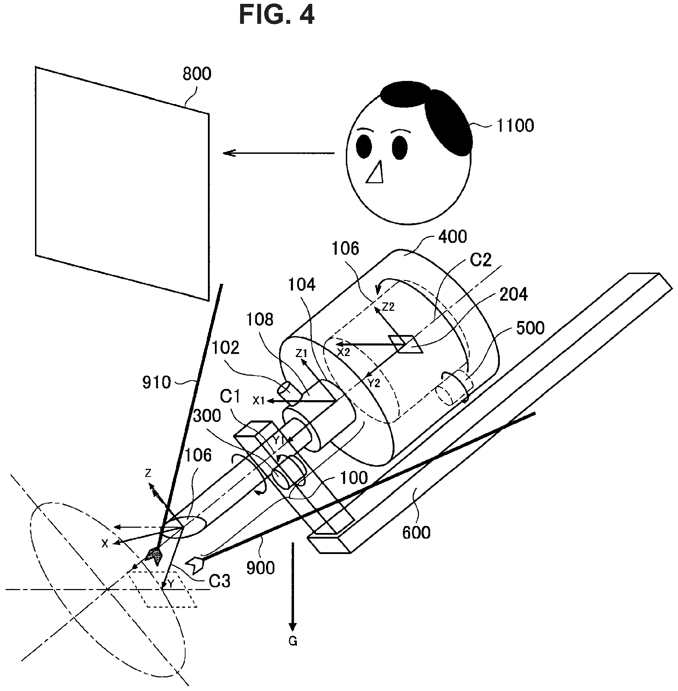

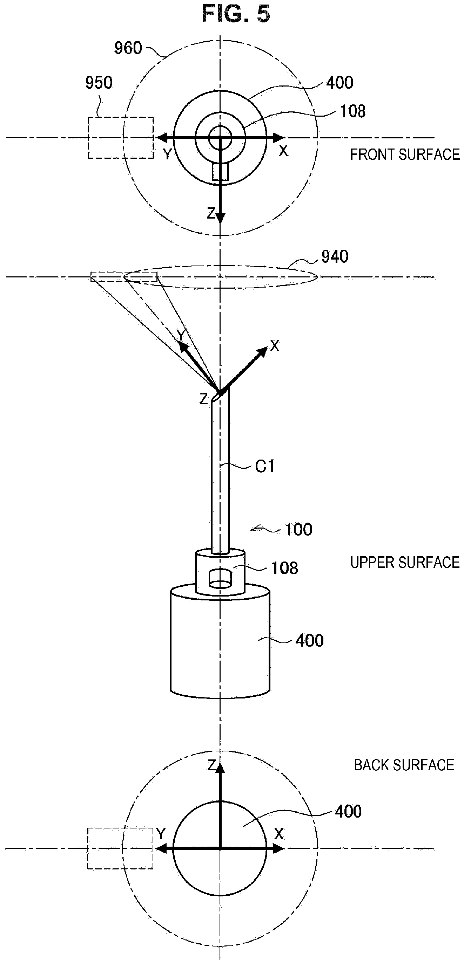

FIG. 4 is a schematic illustration showing a combination of FIG. 1 and FIG. 3. Moreover, FIG. 5 is a schematic illustration showing a positional relationship in the case of viewing from an upper surface, a front surface (target organization side), and a back surface (camera side) of the obliquely-viewing scope 100. In FIG. 5, a flat plane, in which there exists the target organization 930, vertical to the optical axis C1 of the eyepiece optics system, is defined as a surgery flat plane 940, and, on the surgery flat plane 940, a camera visual-field flat plane 950 is indicated. The center of the camera visual-field flat plane 950 will move on a rotation movement locus 960 by the rotation of the obliquely-viewing scope 100.

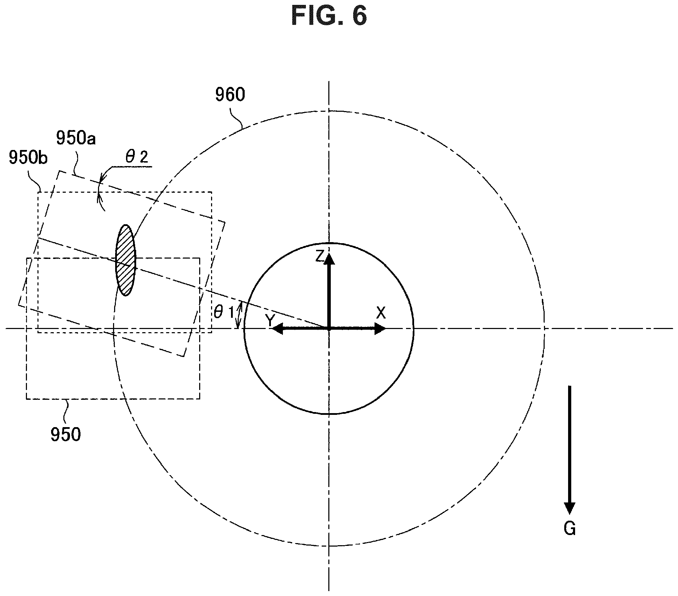

FIG. 6 shows an enlarged illustration of the camera visual-field flat plane 950. In FIG. 6, it is assumed that the position of the camera visual-field flat plane 950 shown in FIG. 5 is made an initial position and, at this time, the target organization 930 is located at the position shown in the illustration.

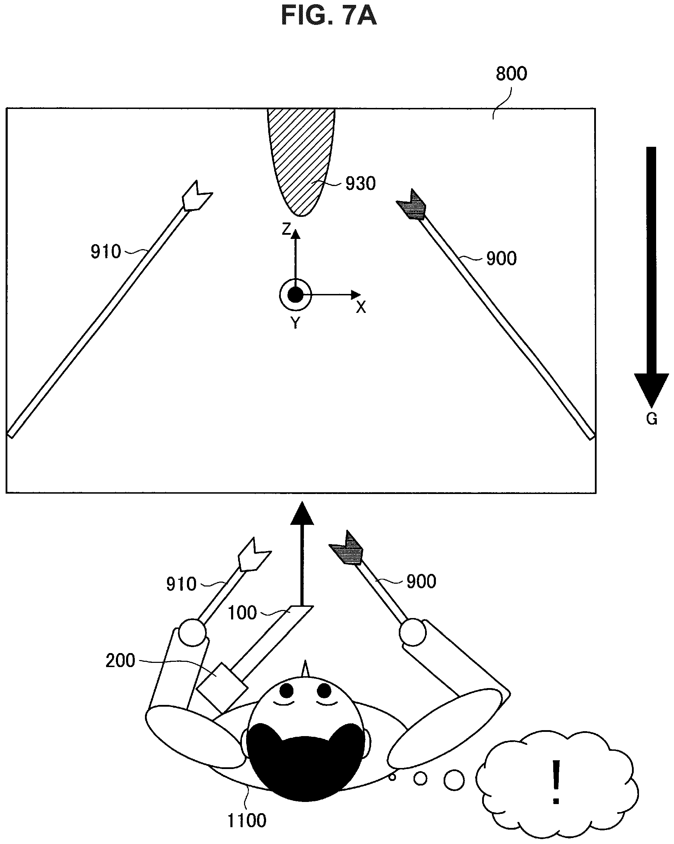

Moreover, FIG. 7A through FIG. 7C each is a schematic illustration showing a scope image displayed on the monitor 800 and the surgeon 1100. FIG. 7A shows the scope image displayed on the monitor 800 at the initial position of the camera visual-field flat plane 950. As shown in FIG. 7A, at the initial position, the target organization 930 is interrupted by the upper side of the screen of the monitor 800. In this state, since the vertical direction of an image on the monitor 800 coincides with the gravity direction, the hand eye coordination is good. However, since the optical axis C3 of the objective optical system is directed to a direction different from the target organization 930, the whole target organization 930 cannot be observed on the screen of the monitor 800.

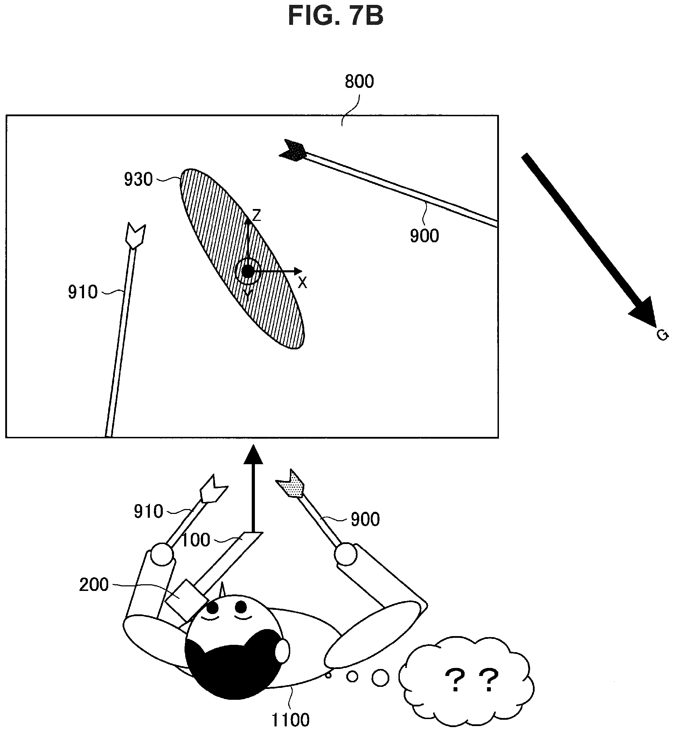

Then, the obliquely-viewing scope 100 is rotated around the optical axis C1, serving as the center of rotation, of the eyepiece optics system by the scope rotating device 300. As mentioned in the above, this rotation may be performed manually. Upon rotating the obliquely-viewing scope 100 with this operation, like the camera visual-field flat plane 950a shown in FIG. 6, the target organization 930 is made to be located at the center of the screen. The screen of the monitor 800 in this state is shown in FIG. 7B. As shown in FIG. 7B, upon rotating the obliquely-viewing scope 100, the gravity direction does not coincide with the vertical direction of the screen, which causes in inclination.

In the state shown in FIG. 7B, the surgeon 1100 cannot maintain the hand eye coordination. Accordingly, it is inevitable to take a countermeasure for making the gravity direction of the target organization 930 coincide with the vertical direction, for example, by inclining the neck relative to the monitor 800, or the like.

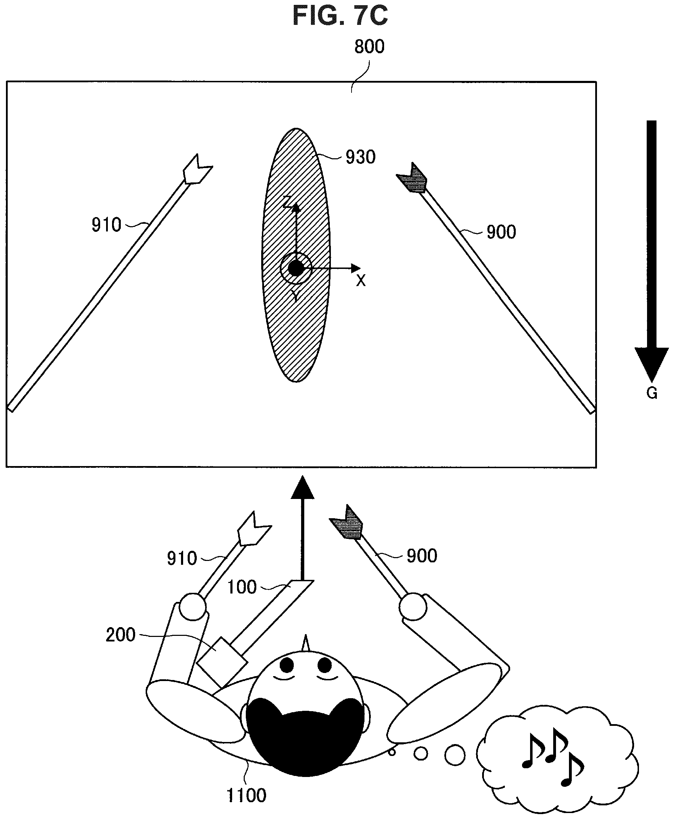

Then, in the present embodiment, the camera 200 is made to rotate relative to the housing 400 by the camera rotating device 500 so as not to change a relative angle between the direction of the gravity G and the Z2 vector. FIG. 7C shows a state where the camera 200 has been made to rotate relative to the housing 400. With this, like the camera visual-field flat plane 950b shown in FIG. 6, in the state where the target organization 930 has been located at the center of the screen of the monitor 800, the gravity direction of the target organization 930 coincides with the vertical direction of the screen. Therefore, as shown in FIG. 7C, it becomes possible for the surgeon 1100 to visually recognize a scope image and to operate the right hand forceps 900 and the left hand forceps 910 with the left hand in the state where the gravity direction of the target organization 930 coincides with the vertical direction of the screen. In the state shown in FIG. 7C, the direction of the gravity in the display coordinate system of the monitor 800 also does not change, and the positional relationship between right and left in the right hand forceps 900 and the left hand forceps 910 on the both left and right hands on the screen of the monitor 800 also does not change. Accordingly, it becomes possible for the surgeon to perform treatment in the state where good hand eye coordination is secured.

### 5. Constitution example of control device

In order to perform the control described in the above, the endoscope system 1000 shown in FIG. 1 includes a control device 150. The control device 150 includes a scope rotation angle acquiring section 152 that acquires the rotation angle of the scope rotating device 300, a gravity direction acquiring section 154 that acquires the direction of gravity, a camera rotation angle calculating section 156 that calculates the rotation angle of the camera rotating device 500 on the basis of the scope rotation angle relative to the direction of gravity, a camera rotation angle control section 158 that controls the camera rotating device 500 on the basis of the rotation angle calculated by the camera rotation angle calculating section 156, an operation input section 160 into which an operation of the surgeon 1100 is input, and a scope rotation angle control section 162 that controls the rotation angle of the scope rotating device 300 on the basis of the operation of the surgeon 1100. In this connection, the constitution elements of the control device 150 shown in FIG. 1 may be constituted by hardware (circuit) or a central processing unit, such as a CPU, and a program for making these function.

The housing 400 is equipped with a gravity sensor 170. The gravity direction acquiring section 154 of the control device 150 acquires the direction of gravity on the basis of the detection value of the gravity sensor 170. The gravity direction acquiring section 154 may acquire the direction of gravity with other techniques, such as model calculation.

The scope rotation angle acquisition 152 acquires the rotation angle of the obliquely-viewing scope 100 with making the direction of gravity criterion. In concrete terms, the scope rotation angle acquisition 152 acquires the rotation angle (an angle θ1 shown in FIG. 6) of the obliquely-viewing scope 100 relative to the horizontal direction (an X1Y1 plane) with making the direction of gravity G shown in FIG. 6 criterion. As mentioned in the above, the scope rotating device 300 includes an angle sensor that detects the rotation angle of a motor. Accordingly, by previously determining the reference position (horizontal direction) relative to the direction of gravity, the scope rotation angle acquisition 152 can detect an angle θ1 relative to the horizontal direction from a detection value of the angle sensor. In this connection, the reference position relative to the gravity direction is not limited to the horizontal direction, and the reference position may be determined in an arbitrary direction.

The camera rotation angle calculating section 156 calculates the rotation angle of the camera rotating device 500 on the basis of the angle θ1 acquired by the scope rotation angle acquisition 152 with making the gravity direction criterion. In concrete terms, the camera rotation angle calculating section 156 calculates the rotation angle (an angle θ2 shown in FIG. 6) of the camera 200 relative to the horizontal direction (an X2Y2 plane) with making the direction of gravity G criterion. As shown in FIG. 6, the angle θ2 is made equal to the angle θ1 (θ1 = θ2).

The camera rotation angle control section 158 controls the camera rotating device 500 on the basis of the rotation angle θ2 calculated by the camera rotation angle calculating section 156.

Moreover, into the operation information input section 160, input is the operation information when the surgeon 1100 has performed an operation to the user interfaces 180, such as an operation button and a touch panel. The surgeon 1100 operates the user interface 180 with reference to an image on the monitor 800, and inputs the amount of rotation of the obliquely-viewing scope 100 as operation information. The scope rotation angle control section 162 controls the rotation angle of the scope rotating device 300 on the basis of the operation information having been input into the operation information input unit 160.

In this connection, as mentioned in the above, the surgeon 1100 may operate the obliquely-viewing scope 100 manually. In this case, the scope rotating device 300 does not need to include a motor, and may be permissible if including a rotation angle sensor that detects the rotation angle of the obliquely-viewing scope 100. Moreover, in the description with regard to FIG. 6 and FIG. 7, since the rotation position of the obliquely-viewing scope 100 is synchronous with the rotation position of the housing 400 as a matter of the constitution shown in FIG. 1, the housing 400 may be fixed to the holding section 600, and the holding section 600 may rotate the housing 400 by the scope rotating device 300. Moreover, the housing 400 may be independent of the rotation position of the obliquely-viewing scope 100. In the constitution shown in FIG. 2, it is possible to perform operation with grasping only the housing 400. If the direction of gravity of the housing 400 is known, the camera 200 is driven relative to the housing 400 by the driving force of the camera rotating device 500. In this case, the camera rotation angle calculating section 156 calculates the rotation angle of the camera rotating device 500 on the basis of "a camera rotation angle" relative to the direction of gravity. The angle θ1 shown in FIG. 6 is only used by the scope rotation angle control section 162 together with the operation information from the surgeon 1100. The camera rotation angle calculating section 156 detects the angle θ2 relative to the horizontal direction from the detection value of the angle sensor of the camera rotating device 500 by previously determining the reference position (the horizontal direction) relative to the gravity direction acquired from the gravity direction acquiring section 154. Finally, the camera rotation angle control section 158 controls the camera rotating device 500 on the basis of the rotation angle θ2 calculated by the camera rotation angle calculating section 156. In this connection, the reference position relative to the gravity direction is not limited to the horizontal direction, and the reference position may be determined in an arbitrary directions.

With the constitution described in the above, even if the vertical direction of the screen of the monitor 800 and the direction of the gravitational acceleration G become a state different from each other correspondingly to the operation for the obliquely-viewing scope 100, it is possible to make the Z2 vector (the vertical direction of the screen of the image sensor 204) and the direction of the gravitational acceleration G coincide with each other relative to the image sensor 204 with making the Y2 vector the center. Accordingly, as a result, it becomes possible to maintain good hand eye coordination. In this connection, in the case where the optical axis C2 of the imaging optical system and the gravitational acceleration G coincide with each other perfectly, since the Z2 vector becomes indefinite, it is desirable not to cause the camera 200 to be rotated.

Moreover, in the example mentioned in the above, as one example, description is given for a case of making the vertical direction of the screen of the monitor 800 and the direction of the gravitational acceleration G coincide with each other. However, for example, as required, such as a case where the surgeon 1100 observes the monitor 800 by inclining the face, the vertical direction (the vertical direction relative to the horizontal plane connecting both eyes) of the head of the surgeon 1100 and the vertical direction (vertical direction of an image displayed on the screen of the monitor 800) of the camera 200 (image sensor 204) may be made to coincide with each other. In this case, it is sufficient if a device such as a head mounted display (HMD) is mounted on the head of the surgeon 1100, the vertical direction of the head is detected by a gravity sensor etc. equipped in the head mounted display, and then, the rotation angle θ2 of the camera 200 is corrected with this detection value.

### 6. Constitution example in case of straightly-viewing scope

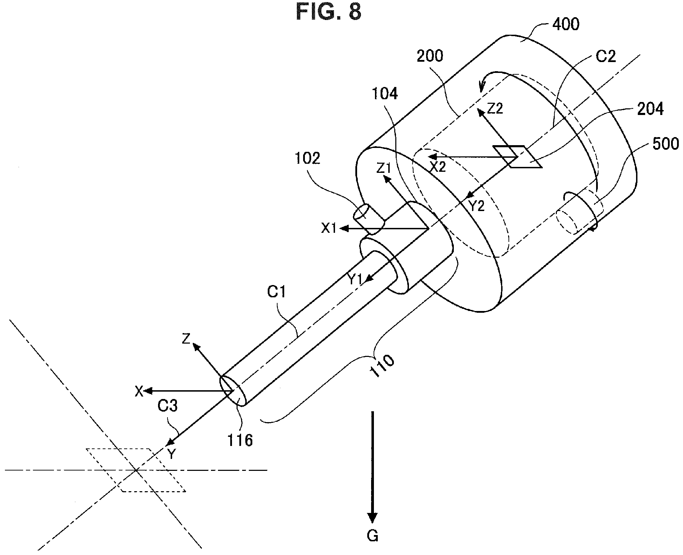

FIG. 8 is a schematic illustration showing a basic constitution in the case of using a straightly-viewing scope 110 instead of the obliquely-viewing scope 100. In the case of the straightly-viewing scope 110, the scope rotating device 300 is eliminated from the constitution of the obliquely-viewing scope 100 shown in FIG. 1 so that the constitution can be made a simple constitution. Also in the constitution shown in FIG. 8, the straightly-viewing scope 110 and the camera 200 are connected so as to make the optical axis C1 of the eyepiece optics system of the straightly-viewing scope 110 and the optical axis C2 of the imaging optical system of the camera 106 coincide with each other.

On the distal end of the straightly-viewing scope 110, the objective lens section 116 is arranged such that the optical axis C1 of the eyepiece optics system and the optical axis C3 of the objective optical system coincide with each other. The camera 200 is stored substantially integrally in the housing 400. The camera 200 is made rotatable freely relative to the housing 400, and is driven relative to the housing 400 by the driving force of the camera rotating device 500.

Similarly to FIG. 2, the housing 400 may be connected to the supporting arm 700. The surgeon 1100 can operate the straightly-viewing scope 110 by grasping the housing 400 directly.

Even if the vertical direction of the screen and the direction of the gravitational acceleration G become a state different from each other by the operation of the surgeon 1100, with these constitutions, it is possible to make the Z2 vector (the vertical direction of the screen of the image sensor 204) and the direction of the gravitational acceleration G coincide with each other easily relative to the image sensor 204 with making the Y2 vector the center. Accordingly, as a result, it is possible to maintain good hand eye coordination.

As described in the above, according to the present embodiment, in the case where the vertical direction of an endoscope image and the vertical direction of an image displayed on the monitor 800 become not to coincide with each other due to the rotation of a scope, the vertical direction of the endoscope image and the vertical direction of the image displayed on the monitor 800 are made to coincide with each other by rotating the camera 200. With this, it becomes possible to maintain good coordination between the visual sense of the surgeon 1100 and the motions of hands. Consequently, it becomes possible to perform surgery easily.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) A control device of an endoscope system, including:
   a scope rotation angle acquiring section that acquires a scope rotation angle, around an axis in an insertion direction, of a scope including an objective lens to be inserted in a body; and
   a rotation angle control section that controls a rotation angle, around an optical axis center, of an image sensor that captures an image provided by the scope, on the basis of the scope rotation angle.
(2) The control device of an endoscope system according to (1), including:
   a rotation angle calculating section that calculates the rotation angle, around an optical axis center, of the image sensor on the basis of the scope rotation angle.
(3) The control device of an endoscope system according to (1) or (2), in which the rotation angle control section controls the rotation angle, around an optical axis center, of the image sensor such that the image sensor rotates by the scope rotation angle.
(4) The control device of an endoscope system according to any of (1) to (3), in which the scope is an obliquely-viewing scope including the objective lens that has an optical axis with a predetermined angle relative to an insertion direction.
(5) The control device of an endoscope system according to any of (1) to (4), in which the scope rotation angle acquiring section acquires the scope rotation angle that makes a gravity direction a criterion.
(6) The control device of an endoscope system according to any of (1) to (5), including:
   a gravity direction acquiring section that acquires the gravity direction on the basis of a detection value of a gravity sensor.
(7) The control device of an endoscope system according to any of (1) to (6), including:
   an operation information input section to which operation information by a user is input, and
   a scope rotation angle control section that controls a rotation angle of a rotating device that rotates the scope around an axis in an insertion direction on the basis of the operation information.
(8) A control method of an endoscope system, including:
   acquiring a scope rotation angle, around an axis in an insertion direction, of a scope including an objective lens to be inserted in a body; and
   controlling a rotation angle, around an optical axis center, of an image sensor that captures an image provided by the scope, on the basis of the scope rotation angle.
(9) A control device of an endoscope system, including:
   a camera rotation angle calculating section that calculates a rotation angle, around an optical axis center, of an image sensor that captures an image provided by a scope including an objective lens to be inserted in a body; and
   a rotation angle control section that controls a rotation angle, around an optical axis center, of the image sensor on the basis of the rotation angle.
(10) A control method of an endoscope system, including:
   calculating a rotation angle, around an optical axis center, of an image sensor that captures an image provided by a scope including an objective lens to be inserted in a body; and
   controlling a rotation angle, around an optical axis center, of the image sensor on the basis of the rotation angle.

### Reference Signs List

- 150: control device
- 152: scope rotation angle acquiring section
- 154: gravity direction acquiring section
- 156: camera rotation angle calculating section
- 158: camera rotation angle control section
- 1000: endoscope system

## Claims

1. A control device of an endoscope system, comprising:
a scope rotation angle acquiring section that acquires a scope rotation angle, around an axis in an insertion direction, of a scope including an objective lens to be inserted in a body; and
a rotation angle control section that controls a rotation angle, around an optical axis center, of an image sensor that captures an image provided by the scope, on a basis of the scope rotation angle.

2. The control device of an endoscope system according to claim 1, comprising:
a rotation angle calculating section that calculates the rotation angle, around an optical axis center, of the image sensor on a basis of the scope rotation angle.

3. The control device of an endoscope system according to claim 1, wherein the rotation angle control section controls the rotation angle, around an optical axis center, of the image sensor such that the image sensor rotates by the scope rotation angle.

4. The control device of an endoscope system according to claim 1, wherein the scope is an obliquely-viewing scope including the objective lens that has an optical axis with a predetermined angle relative to an insertion direction.

5. The control device of an endoscope system according to claim 1, wherein the scope rotation angle acquiring section acquires the scope rotation angle that makes a gravity direction a criterion.

6. The control device of an endoscope system according to claim 5, comprising:
a gravity direction acquiring section that acquires the gravity direction on a basis of a detection value of a gravity sensor.

7. The control device of an endoscope system according to claim 1, comprising:
an operation information input section to which operation information by a user is input, and
a scope rotation angle control section that controls a rotation angle of a rotating device that rotates the scope around an axis in an insertion direction on a basis of the operation information.

8. A control method of an endoscope system, comprising:
acquiring a scope rotation angle, around an axis in an insertion direction, of a scope including an objective lens to be inserted in a body; and
controlling a rotation angle, around an optical axis center, of an image sensor that captures an image provided by the scope, on a basis of the scope rotation angle.

9. A control device of an endoscope system, comprising:
a camera rotation angle calculating section that calculates a rotation angle, around an optical axis center, of an image sensor that captures an image provided by a scope including an objective lens to be inserted in a body; and
a rotation angle control section that controls a rotation angle, around an optical axis center, of the image sensor on a basis of the rotation angle.

10. A control method of an endoscope system, comprising:
calculating a rotation angle, around an optical axis center, of an image sensor that captures an image provided by a scope including an objective lens to be inserted in a body; and
controlling a rotation angle, around an optical axis center, of the image sensor on a basis of the rotation angle.
